**Europäisches Patentamt**

(19) **European Patent Office**

**Office européen des brevets**

(11) Veröffentlichungsnummer: **0 261 186 B1**

(12) **EUROPÄISCHE PATENTSCHRIFT**

(45) Veröffentlichungstag der Patentschrift :
05.06.91 Patentblatt 91/23

(51) Int. Cl.⁵ : **A61F 2/16**

(21) Anmeldenummer: **87902065.9**

(22) Anmeldetag : **01.04.87**

(86) Internationale Anmeldenummer :
**PCT/DE87/00142**

(87) Internationale Veröffentlichungsnummer :
**WO 87/05797 08.10.87 Gazette 87/22**

(54) **INTRAOKULARE IMPLANTATIONSLINSE.**

(30) Priorität : 01.04.86 DE 3610833

(43) Veröffentlichungstag der Anmeldung :
30.03.88 Patentblatt 88/13

(45) Bekanntmachung des Hinweises auf die
Patenterteilung :
05.06.91 Patentblatt 91/23

(84) Benannte Vertragsstaaten :
**DE FR GB NL**

(56) Entgegenhaltungen :
**EP-A- 0 064 770**
**EP-A-01 617 65**
**WO-A-60/3961**

(56) Entgegenhaltungen :
**DE-A- 2 723 883**
**DE-A-27 589 12**
**DE-A-34 288 95**
**US-A-42 778 52**

(73) Patentinhaber : **INPROHOLD Establishment**
**Schwefelstrasse 33**
**FL-9490 Vaduz (LI)**

(72) Erfinder : **SCHLEGEL, Hans-Joachim**
**Siebenpfeifferstrasse 22**
**W-6650 Homburg/Saar (DE)**

(74) Vertreter . **Wey, Hans-Heinrich, Dipl.-Ing.**
**Patentanwälte Wey & Partner**
**Widenmayerstrasse 49**
**W-8000 München 22 (DE)**

## Beschreibung

Die Erfindung betrifft eine flexible intraokulare Implantationslinse als Ersatz für die aus dem Auge von Lebewesen höherer Ordnung operativ, insbesondere extrakapsular entfernte natürliche Linse.

Implantationslinsen der vorbezeichneten Art sind Gegenstand des Europäischen Patents 064 770 und sind somit aus der EP-A-0 064 770 bekanntgeworden. Sie haben sich bereits in der ophthalmologischen Praxis hervorragend bewährt. Diese Linsen besitzen einen zentralen, als Sammellinse ausgebildeten Linsenkörper mit sich von diesem peripher radial nach außen erstreckenden und diesen in seiner Lage fixierenden Halterungen in Form dünnwandiger flächiger Stützelemente, deren äußerer Rand auf einem Kreisbogen um den Mittelpunkt des Linsenkörpers liegt, der sich entweder im Linsenkapselsack oder in der Ziliarfurche abstützt, und welche aus einem homogenen, glasklaren, hochtemperaturbeständigen Kunststoff, vorzugsweise aus vulkanisiertem Silikonwerkstoff bestehen, welcher ein spezifisches Gewicht zwischen 0,01 und 1,08, vorzugsweise von etwa 1,02, besitzt.

Aus der EP-A-0 180 887 ist es bekannt, Linsen der vorerwähnten Art in der Weise auszubilden, daß wenigstens eine der beiden Oberflächen des zentralen Linsenkörpers in der Art der optisch wirksamen Fläche einer Fresnel-Linse ausgebildet ist, um deren Faltbarkeit zum Zwecke der Implantation positiv zu beeinflussen.

Diese Linsen dienen als Ersatz für die aus dem Auge von Lebewesen, insbesondere von Menschen, infolge einer durch Katarakt getrübten, operativ entfernten Linse. Derartige Kunstlinsen sind in der Regel glasklar und für Strahlen des sichtbaren Lichts mit einer Wellenlänge von etwa 400 bis etwa 800 nm sowie auch für Strahlen des ultravioletten Bereichs mit einer Wellenlänge von etwa 280 bis etwa 400 nm durchlässig.

Ältere bzw. greise Patienten, welchen eine solche intraokulare Linse implantiert worden ist, klagen häufig über postoperative Blendungsbeschwerden, was durchaus verständlich ist, da die operativ entfernte natürliche Linse eine altersbedingte Gelbfärbung besaß, während die implantierte Linse glasklar ist.

Der Erfindung liegt somit die Aufgabe zugrunde, intraokulare Implantationslinsen der in Betracht kommenden Art, welche aus einem elastischen, flexiblen Material bestehen, das sich als biokompatibel und chemisch inert erwiesen hat, und auch faltbar sind, zu verbessern und weiterzuentwickeln, damit sie bei Aufrechterhaltung der Transparenz in farblicher Hinsicht derart eingestellt sind, daß sie in etwa derjenigen des gesunden, gealterten Auges entsprechen, ohne daß Komplikationen für das Auge, in welches eine solche Linse implantiert worden ist, zu befürchten sind. Weiterhin sollen solche Linsen auch so ausgebildet sein, daß sie in der Lage sind, ultraviolette Strahlen zumindest in einem als notwendig erachteten Ausmaß zu absorbieren.

Zur Lösung dieser Aufgabe wird gemäß der Erfindung vorgeschlagen, die intraokulare Implantationslinse in der Weise auszubilden, daß in den Werkstoff der Linse eine transparente, in gelblich-bräunlichem Farbton, der in etwa dem der natürlichen Linse infolge fortschreitender Vergilbung entspricht, eingefärbte Scheibe aus einem flexiblen Kunststoff eingebettet ist, welche von dem die Linse bildenden Werkstoff vollständig umgeben ist, so daß sie an keiner Stelle mit dem die Linse umgebenden Augenkammerwasser in Berührung kommen kann. Auf diese Weise wird erreicht, daß die altersgemäße Farbempfindung des operierten Auges wiederhergestellt wird, so daß diese in etwa derjenigen eines gesunden Auges entspricht.

Würde man die für die angestrebte Einfärbung der Linsen benutzten Substanzen dem Werkstoff, aus welchem sie bestehen, beimischen, so hätte dies den wesentlichen Nachteil, daß die in Betracht kommenden Substanzen zwar homogen im Molekulargefüge des Kunststoffmaterials verteilt, jedoch auch an der Oberfläche der Linsen dem Einfluß des Augenkammerwassers ausgesetzt sind, so daß unerwünschte chemische bzw. biochemische Reaktionen stattfinden können und auch ein Herauslösen dieser Substanzen aus dem Molekulargefüge des Werkstoffs möglich ist.

Nachdem aber auch die Einwirkung ultravioletter Strahlen auf die Retina unerwünscht ist, wird ferner insbesondere ergänzend vorgeschlagen, die in den Werkstoff der Linse eingebettete Kunststoffscheibe zusätzlich für UV-Strahlen zumindest weitestgehend undurchlässig zu machen.

Man hat bereits dem Werkstoff von PMMA-Implantationslinsen UV-Strahlen absorbierende Substanzen beigemischt, so daß hinsichtlich solcher als auch wie vorerwähnt eingefärbter Linsen die vorerwähnten Mängel in besonderem Maß zu befürchten sind. Selbst wenn bei bisher kurzfristigen Beobachtungen derartige Feststellungen noch nicht getroffen werden konnten, so muß man erfahrungsgemäß mit solchen Prozessen auf lange Sicht dennoch rechnen. Die Erfahrungen der Implantationschirurgie am Auge lehren, daß Substanzen, die man ursprünglich für biochemisch inert und deshalb für unzerstörbar hielt, wie z.B. Polyamide oder Polypropylen oder dgl., im Laufe der Zeit innerhalb des Auges verrotteten und darüber hinaus zu toxischen Abbauprodukten wurden. Dies hatte die Konsequenz, daß eine beträchtliche Anzahl solcher Implantationslinsen aus operierten Augen wieder haben entfernt werden müssen. Hieraus resultiert die Feststellung, daß es außerordentlich gefährlich ist, in das Auge von Lebewesen solche Linsen zu implantieren, die sich nicht chemisch und biochemisch absolut neutral verhalten.

Wie aus der DE-A-34 28 895 bekanntgeworden ist, ist es bereits vorgeschlagen worden, bei einer "Doppellinse", welche aus einem bikonvexen Linsenkern aus einem Polyimidkunststoff besteht, welcher von einem Silikonmantel allseitig umschlossen ist, den bikonvexen Linsenkern einzufärben und mit einem Antireflexbelag zu versehen. Diese "Doppellinse" ist jedoch eine solche einer ganz anderen Gattung als diejenigen Linsen der vorliegend in Betracht kommenden Art.

Geht man wie erfindungsgemäß vorgeschlagen vor, erhält man flexible, falt- bzw. einrollbare Linsen, die sich für eine spezielle zukunftsträchtige Operationsmethode besonders gut eignen und hinsichtlich welcher eine absolute Verträglichkeit gewährleistet ist.

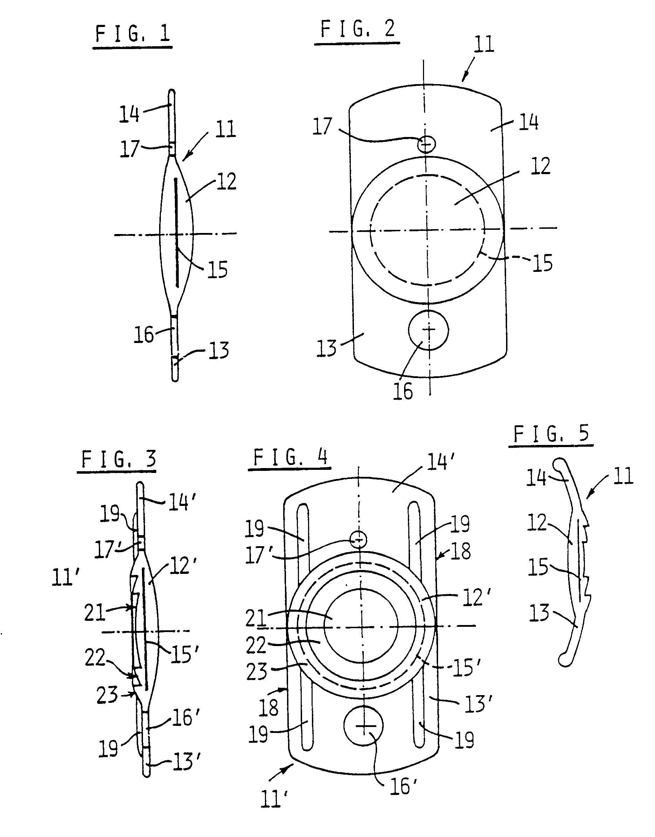

Weitere Merkmale der erfindungsgemäß ausgebildeten Implantationslinse gehen aus den Unteransprüchen und der nachstehenden Beschreibung zweier bevorzugter, in den Figuren 1 bis 5 der Zeichnung dargestellter Ausführungsbeispiele hervor, welche nachstehend im einzelnen näher beschrieben sind. ES zeigen :

Fig. 1 einen Längsschnitt durch eine Implantationslinse mit bikonvexem Linsenköper, in deren Werkstoff eine eine strahlenabsorbierende Substanz enthaltende dünne Kunststoffscheibe eingebettet ist ;

Fig. 2 eine Aufsicht auf die Implantationslinse nach Fig. 1 ;

Fig. 3 einen Längsschnitt durch eine Implantationslinse, deren Linsenkörper als modifizierte Fresnel'sche Ringlinse ausgebildet ist ;

Fig. 4 eine Aufsicht auf die Linse nach Fig. 3 ;

Fig. 5 einen Längsschnitt durch eine Implantationslinse mit gekrümmten Stützlappen.

Die in den Fig. 1 und 2 dargestellte Linse 11 ist eine solche von im wesentlichen bekannter Form. Sie besitzt einen zentralen bikonvexen Linsenkörper 12, von welchem sich diametral einander gegenüberliegend radial nach außen bzw. nach unten und oben Stützlappen 13 bzw. 14 erstrecken, die eine geringe, aber ausreichend bemessene Stärke aufweisen, um die implantierte Linse 11 im Auge in ihrer Lage zu halten. Diese Linse 11 besteht vorteilhafterweise aus einem vulkanisierten Silikon-Werkstoff mit einer Shore-Härte von etwa 30 bis 50, vorzugsweise jedoch etwa 35 bis 45, so daß sie eine gute Elastizität und Flexibilität bei ausreichender Stabilität besitzt.

(Weiter Seite 5 oben der ursprünglichen Beschreibung).

In den Werkstoff der Linse 11, und zwar im zentralen Bereich des Linsenkörpers 12, ist eine flexible Scheibe 15 eingebettet, die vorzugsweise gleichfalls aus vulkanisiertem Silikon-Werkstoff oder gegebenenfalls aus einem mit diesem chemisch verwandten Werkstoff besteht, welcher einerseits einen vorzugsweise gelblich-bräunlichen Farbstoff und andererseits eine UV-Strahlen im Bereich von etwa 280 nm bis etwa 400 nm absorbierende Substanz enthält. Diese Scheibe 15 ist zweckmäßigerweise in der Mittenebene der Implantationslinse 11 oder in Lichtdurchtrittsrichtung etwas hinter dieser angeordnet ; letzteres ist für den Strahlengang und die Abschirmung vorteilhafter, da die Scheibe 15 einen Durchmesser haben muß, der etwas kleiner, und zwar vorteilhafterweise um etwa 10 bis 15% kleiner, ist als der Durchmesser des zentralen, optisch wirksamen Linsenkörpers 12.

Im unteren Stützlappen 13 befindet sich eine Öffnung 16, die unterschiedlichsten Zwecken dient, insbesondere kann durch sie Augenkammerwasser hindurchtreten. Im oberen Stützlappen 14 befindet sich eine relativ kleine Öffnung 17, der die Aufgabe zukommt, die Spitze eines bei der Implantation benutzten Einführungsinstruments aufzunehmen, durch welches die konventionelle Operation vereinfacht und erleichtert wird.

Die Linse 11' nach den Fig. 3 und 4 entspricht praktisch derjenigen nach den Fig. 1 und 2. Sie unterscheidet sich von diesen lediglich dadurch, daß der zentrale Linsenkörper 12' als Fresnel'sche Ringlinse ausgebildet ist, und zwar vorliegend beispielsweise als einseitige dreistufige Fresnel-Linse, bei welcher die Stufen vorzugsweise auf der Rückseite des Linsenkörpers 12' angeordnet sind. Radial außerhalb der zentralen, konvex gekrümmten Fläche 21 befinden sich die ringförmigen Linsenstufen 22 und 23 mit entsprechend konvex gekrümmter Oberfläche.

Die zuvor beschriebenen, elastischen, flexiblen Implantationslinsen 11 bzw. 11' bieten den Vorteil, für das Einsetzen in das Auge leicht und mühelos falt- bzw. auch einrollbar zu sein und so bei der Operation nur einen ungewöhnlich kleinen Schnitt von ca. 3 bis 4 mm Länge zu benötigen. Eine Implantationslinse des zweiten Typs nach Fig. 3 und 4 ist diesbezüglich vorteilhafter, da sie im zentralen, optisch wirksamen Bereich 12' nicht die Dicke einer bikonvexen Linse 12 aufweist, so daß ihre Faltbarkeit bzw. Einrollbarkeit besonders gut ist.

Um die Stützlappen 13 und 14 bzw. 13' und 14' in der Längsrichtung gleichermaßen falt- bzw. rollbar, wie in der Querrichtung ausreichend knicksteif auszubilden, sind, wie die Fig. 3 und 4 beispielhaft zeigen, an den Stützlappen 13' und 14' parallel zu den beiden geradlinigen Seitenkanten 18 der Linse 11' zwei wulstartige Versteifungsrippen 19 angeordnet. Diese können entweder nur auf der Vorderseite oder nur auf der Rückseite der Linse 11' oder aber auch auf beiden Seiten vorgesehen sein. In entsprechender Weise lassen sich diese Rippen 19 bei der Linse 11 nach den Fig. 1 und 2 vorsehen.

Die erfindungsgemäß ausgebildete Implantationslinse kann vorzugsweise auch eine Form haben, sie sie aus Fig. 5 beispielsweise ersichtlich ist.

## Ansprüche

1. Intraokulare Implantationslinse (11) als Ersatz für die aus dem Auge von Lebewesen höherer Ordnung operativ, insbesondere extrakapsulär entfernte natürliche Linse, welche

a) einen zentralen, als Sammellinse ausgebildeten Linsenkörper (12),

b) an dem Linsenkörper angeordnete, sich von diesem peripher radiat nach außen erstreckende und diesen zentrierende Halterungen in Form von Stützlappen (13, 14) aufweist,

c) aus einem homogenen, glasklaren Kunststoff, vorzugsweise aus vulkanisiertem Silikonwerkstoff, besteht,

d) zum Zwecke der Implantation faltbar ist, dadurch gekennzeichnet, daß in den Werkstof des Linsen Körpers (12) eine transparente, in gelblich-bräunlichem Farbton eingefärbte Scheibe (15) aus flexiblem Kunststoff eingebettet ist.

2. Implantationslinse nach Anspruch 1, dadurch gekennzeichnet, daß die in den Werkstoff der Linse (11) eingebettete Scheibe (15) aus dem gleichen oder einem chemisch verwandten Werkstoff wie die Linse selbst besteht.

3. Implantationslinse insbesondere nach Anspruch 1 oder 2, dadurch gekennzeichnet, daß der Werkstoff der in die Linse (11) eingebetteten Scheibe (15) eine ultraviolette Strahlen mit einer Wellenlänge von etwa 280 nm bis etwa 400 nm wenigstens weitestgehend absorbierende Substanz enthält.

4. Implantationslinse nach Anspruch 1, 2 oder 3, dadurch gekennzeichnet, daß die Scheibe (15) in Richtung des Lichtdurchtritts hinter der Mittenebene der Linse (11) angeordnet ist.

5. Implantationslinse nach einem oder mehreren der Ansprüche 1 bis 4, dadurch gekennzeichnet, daß die Scheibe (15) einen um etwa 10 bis 15% kleineren Durchmesser besitzt als der zentrale, optisch wirksame Linsenkörper (12).

6. Implantationslinse nach einem oder mehreren der Ansprüche 1 bis 5, dadurch gekennzeichnet, daß der Linsenkörper (12) eine bikonvexe Sammellinse ist.

7. Implantationslinse nach einem oder mehreren der Ansprüche 1 bis 5, dadurch gekennzeichnet, daß der Linsenkörper (12') eine zwei, drei- oder gegebenenfalls mehrstufige Fresnel'sche Ringlinse ist.

## Claims

1. An intra-ocular implant lens (11) as a replacement for the natural lens surgically, particularly extracapsularly, removed from the eye of living beings of a higher order,

a) having a central lens body (12) provided as a collecting lens, and

b) holders in the form of support tabs (13, 14), arranged on said lens body, extending radially outwardly from the periphery thereof and serving to centre said lens body,

c) consisting of a homogenous, crystal-clear plastic, preferably a vulcanized silicone material,

d) and being foldable for the implantation, characterized in that a transparent disc (15) tinted to a yellowish-brown shade of colour and consisting of flexible plastic is embedded in the material of said lens body (12).

2. An implant lens according to claim 1, characterized in that the disc (15) embedded in the material of the lens (11) consists of a material the same as or chemically related to that of the lens itself.

3. An implant lens particularly according to claim 1 or 2, characterized in that the material of the disc (15) embedded in the lens (11) contains a substance substantially absorbing ultraviolet rays with a wavelength of approximately 280 nm to approximately 400 nm.

4. An implant lens according to claim 1, 2 or 3, characterized in that the disc (15) is located behind the central plane of the lens (11) in the direction of light passage.

5. An implant lens according to one or more of claims 1 to 4, characterized in that the disc (15) is approximately 10 to 15% smaller in diameter than the central, optically effective lens body (12).

6. An implant lens according to one or more of claims 1 to 5, characterized in that the lens body (12) is a biconvex collecting lens.

7. An implant lens according to one or more of claims 1 to 5, characterized in that the lens body (12') is a one-step, two-step or, where applicable, multistep annular Fresnel lens.

## Revendications

1. Lentille d'implantation intraoculaire (11) remplaçant la lentille naturelle extraite par opération, notamment extracapsulaire, de l'oeil d'êtres vivants supérieurs, lentille d'implantation qui

a) possède un corps lenticulaire (12) central en forme de lentille convergente,

b) possède des fixations en forme de languettes d'appui (13, 14), disposées sur la périphérie du corps lenticulaire et dirigées radialement vers l'extérieur et centrés sur celui-ci,

c) est constituée d'une matière synthétique homogène limpide, préférentiellement d'un matériau de silicone vulcanisée,

d) est flexible aux fins d'implantation, caractérisée par le fait qu'un disque transparent (15), coloré dans une nuance de coloris jaunâtre-brunâtre et en matière synthétique flexible, est noyé dans le matériau du corps lenticulaire (12).

2. Lentille d'implantation selon revendication 1, caractérisée par le fait que le disque (15) noyé dans le matériau de la lentille (11) est constitué d'un matériau identique ou chimiquement proche de celui constituant la lentille elle-même.

3. Lentille d'implantation essentiellement selon revendications 1 ou 2, caractérisée par le fait que le matériau du disque (15) noyé dans la lentille (11) contient une substance absorbant au moins le plus possible des radiations ultraviolettes de longueur d'onde d'environ 280 nm à environ 400 nm.

4. Lentille d'implantation selon revendications 1, 2 ou 3, caractérisée par le fait que le disque (15) est disposé en arrière du plan médian de la lentille (11) dans la direction de pénétration de la lumière.

5. Lentille d'implantation selon une ou plusieurs des revendications 1 à 4, caractérisée par le fait que le disque (15) possède un diamètre d'environ 10 à 15% inférieur à celui du corps central, optiquement efficace, de la lentille.

6. Lentille d'implantation selon une ou plusieurs des revendications 1 à 5, caractérisée par le fait que le corps de lentille (12) est une lentille convergente biconvexe.

7. Lentille d'implantation selon une ou plusieurs des revendications 1 à 5, caractérisée par le fait que le corps de lentille (12') est une lentille annulaire selon Fresnel à deux, trois, ou le cas échéant à plusieurs degrés.

FIG. 1

FIG. 2

FIG. 3

FIG. 4

FIG. 5

6